# EUROPEAN PATENT SPECIFICATION

(11) **EP 2 262 517 B1**
(45) Date of publication and mention of the grant of the patent: **28.11.2012**
(21) Application number: 09717881.8
(22) Date of filing: 16.02.2009
(51) Int. Cl.: A61K 36/81

(54) **METHOD OF MEDICINAL TOBACCO OIL PRODUCTION**
VERFAHREN ZUR HERSTELLUNG VON MEDIZINISCHEM TABAKÖL
PROCÉDÉ DE PRODUCTION D HUILE DE TABAC MÉDICINALE

(30) Priority: 05.03.2008 KZ 20080252
(43) Date of publication of application: 22.12.2010
(73) Proprietor: Murzagaliyev, Abat, Almaty 050000 (KZ); Chebotova, Alla Nicolaevna, Almaty oblast 040622 (KZ)
(72) Inventor: Murzagaliyev, Abat, Almaty 050000 (KZ); Chebotova, Alla Nicolaevna, Almaty oblast 040622 (KZ)
(74) Representative: Jeck, Anton
(86) International application number: PCT/KZ2009/000003
(87) International publication number: WO 2009/110775

(56) References cited:
- EP-A- 0 081 231
- WO-A-2007/114679

## Description

### TECHNICAL FIELD

This invention concerns the method of oils production from tobacco raw material and may be used to producing fat oils from high-oleic wastes of tobacco fields, i.e., tobacco seeds.

### BACKGROUND ART

A known method of tobacco oil production from tobacco seeds includes seed cleaning, detoxication and pressing with oil recovery, therefore double pressing is completed, when cleaned seeds are supplied for the first pressing to produce tobacco oil conserving its natural properties and oil cake then delivered for detoxication, while the second pressing makes it possible to produce commercial tobacco oil (RU 2002/0826.1 A). Above mentioned method providing no high cleaning degree, so, produced oil may not be used for medical purposes.

A known method of vegetable oil production from high-oleic raw materials includes seed cleaning, hulling, funning, grinding, crushed seeds pressing and oil recovery.

Tobacco seeds are used as vegetable raw materials which after being funned are detoxified with 33% sodium hydroxide solution at the rate of 4 liters per 100 kg of seeds (RU 2000/1208.1 A). Disadvantage of this method consists in lower oil cleaning degree.

The nearest equivalent to the proposed invention is the method of medical tobacco oil production from tobacco seeds which includes tobacco seeds cleaning, pressing with oil recovery, separation into liquid and solid fractions, then obtained liquid fraction settling at least during three days and its triple filtration carried out to producing medical tobacco oil (RU 2004/0851.4 A).

Liquid fraction settling at least during three days makes this process very complicated. Besides, prolonged liquid fraction settling leads to oil saturation with toxic components extracted from the residue. Also, triple filtration is one more process complicating factor resulting in substantial losses of desired product.

SU 1513021 discloses a method of producing a medical dive oil, where a treatment with achivated chavcoal at a temperature of 160 to 170° C is carried out.

### DISCLOSURE OF INVENTION

This invention is aimed at developing method of oil production from high-oleic wastes of tobacco fields to be used for medical purposes.

Achievable result consists in simplified processing method and reduced degree of toxic components extraction from the residue through reduced settling time and minimized losses of desired product.

The above-mentioned results in a method of producing tobacco oil for medical use, which is achieved through a process which includes tobacco seeds cleaning,
pressing, and separation into liquid and solid fractions and obtained liquid fraction settling with subsequent oil recovery followed by oil filtration conforming to this invention, where obtained liquid fraction is settled during 12-18 hours; before oil filtration it is pre-cleaned with activated coal additions at ratio of oil : coal = 100 : 2-5; oil is heated up to 50 - 60 ° C and exposed to these temperatures for 40-60 minutes; then single filtration is carried out.

Liquid fraction settling during 12-18 hours (instead 72 hours conforming to prototype) substantially simplifies processing method and leads to reduced degree of toxic components extraction from the residue. Liquid fraction settling during less than 12 hours results in incomplete oil recovery, while liquid fraction settling during more than 18 hours leads to oil contamination with toxic components. Single filtration simplifies processing method and minimizes losses of desired product.

Quantitative ratio of oil : coal = 100 : 2-5, heating up to 50-60 °C and exposure to these temperatures for 40-60 minutes provide optimal conditions of oil cleaning and make it possible to produce high quality medical product.

### THE MODES FOR CARRYING OUT THE INVENTION

This process is implemented as follows:

Example 1. Harvested tobacco seeds are cleaned by screening them through sieves of 6-8 mm meshes and compressed under pressure applied to the press in accordance with standard technical specifications so that they are separated into solid (cake) and liquid (oil) fractions. Crude tobacco oil produced under pressure is settled during 12 hours, hi process of settling solid particles are deposed onto the reservoir bottom forming oil sludge (slush), the oil leaving over. Upper layer is separated from the slush and transported into thick-walled enamelware, being added with activated coal at ratio of oil : coal = 100 : 3. Oil-activated coal mixture is heated up to 60 °C and exposed at this temperature during 40 minutes, then, the oil is cooled down to the room temperature and cleaned. Cleaning process consists in filtration through paper filters of medium-density. Cleaned oil is transported into 1-5 -litres amber glass bottles in which it is stored to be packed for consumer.

Proposed process allows reducing settling time and completely adsorbing available impurities with activated coal additions, the filtration process duration being minimized by several times.

Medical tobacco oil shows such organoleptic and chemophysical characteristics as follows:
Appearance Transparent homogeneous liquid without sediment Color From light - up to dark yellow
Odour Odourless
Taste Bitter pH value (pH) Neutral
Acid number, mg, KOH/g 3,6 Content of mechanical impurities Absence
Toxic residue:
HCCH (Hexachlorocyclohexane) and its isomers Absence
DDT (dichloro-diphenyl- trichloroethane) and its metabolites Absence
Aflotoxine BI, mg/kg Absence
Lead, mg/kg 0,025
Cadmium, mg/kg Absence
Arsenic, mg/kg Absence
Mercury, mg/kg Absence
Nicotine Absence
Toxicity level, class 4 (low - hazard products) Skin irritant action Absence
Fatty acids entering into the composition of oil glycerides, %:
Capronic C₆HₙO₂ 0,001
Laurie Ci₂H₂4O₂ 0,003 Palmitic Ci₆H₃₂O₂ 8,927
Stearic C₁₈H₃₆O₂ 7,555
Oleic C₁₈H₃₄O₂ 9,803
Linoleic C₁₈H₃₂O₂ 71,159
Linolenic Ci₈H₃₀O₂ 0,057 Arachidonic C₂oH₃₂O₂ 0,666
Behenic C₂₂H₄₄O₂ 0,293
Other acids 1,536

Medical oil produced conforming to this proposed method may be used as solvent of medical products for peroral administration and external use as well as separate medical product. Up to 5 liters of cleaned and suitable oil are produced from 100 kg of tobacco seeds.

Example 2. Process is carried out conforming to Example 1, while a liquid fraction is settled during 18 hours and activated coal is added into the oil at ratio of oil : coal = 100 : 5. Activated coal-oil mixture is heated up to 50 °C and exposed at this temperature during 60 minutes, then, it is filtered.

### INDUSTRIAL APPLICABILITY

Produced tobacco oil is notable for its high-power antiseptic and antibacterial effects on practically all types of aerobic and anaerobic infections, with marked antivirus, antifungal, anesthetic and deodorant actions.

This oil contains 71.882 % of essential fatty acids, namely, linoleic acid, linolenic acid, arachidonic acid, not synthesized by human organism but whose lack in the food leads to disbolism and has adverse effects on central nervous system activity. Daily human needs of above-mentioned acids reach 2 g. Tobacco oil contains phosphatides, as it was found, composed by 50-60 % of lecithines, effectively involved in general metabolism, enhancing fatty acids utilization and preventing bacony liver, stimulating secretion of pancreatic juice and improving amylase activity.

Tobacco oil monitors human cells regeneration through skin integument, suppresses foreign viruses in oral cavity and nasal chamber. It may be used in treatment of otitis, adenoid disease, anginas, chronic maxillary sinusitis and proved to be very effective in treatment of acute respiratory diseases. Moreover this oil kills the pains, decongests edemas, and resuscitates human functions in cases of neuralgia, myalgia, arthritises and bursitises. It is recommended for treatment of physical injuries, decubituses, frostbites, bums, traumas, grazes etc. Tobacco oil is effective antiacneic preparation, removes furunculosis, visage herpes and genitals. For this purpose it is sufficient to swab out affected skin sections with oil-wetted tampon three-five times a day.

Itch mite perishes after single application of tobacco oil. Interdigital fungus dermatitis is successfully treated with oil-wetted cotton-gauze turundas applied for 10 minutes one-two times a day.

Oil produced by proposed method is effective deodorant and proved to be effective antiperspirant product to treating foot, hand and axillary creases due to intensive odour removing and adverse microflora suppressing. Tobacco oil is used in therapy of hemorrhoids at all stage of disease where anal fissures are greased with oil-wetted tampon or treated with 15-20 ml microclysters. Tobacco oil spraying or oil-wetted tampons are used in therapy of gynecology diseases where 10-15 procedures are sufficient for one course of treatment.

The otitis is treated with tobacco oil wetted turundas applied into the ear 5-6 times a day; adenoid diseases treatment requires to greasing tonsils 3-4 times a day and in cases of chronic maxillary sinusitis 1-2 nasal oil drops are administrated 3-4 times a day.

## Claims

1. A method of producing tobacco oil for medical use which includes tobacco seeds cleaning, pressing, and separation into liquid and solid fractions, obtained liquid fraction settling with subsequent oil recovery followed by oil filtration **characterized by** that the obtained liquid fraction is settled during 12-18 hours and before oil filtration it is pre-cleaned with activated coal additions at ratio of oil : coal = 100 : 2- 5, the oil being heated up to 50-60 °C and exposed to these temperatures for 40-60 minutes; then a single filtration is carried out.

## Patentansprüche

1. Verfahren zur Herstellung von Tabaksamenöl für medizinische Zwecke, wobei das Verfahren das Reinigen von Tabaksamen, deren Pressen und Trennen in Flüssigkeit und feste Fraktionen sowie das Absetzen der gewonnenen Flüssigkeitsfraktion mit einer folgenden Ölfilterung umfasst, der eine Ölrückgewinnung folgt,
**dadurch gekennzeichnet,**
**dass** die gewonnene Flüssigkeitsfraktion 12-18 Stunden lang abgelagert und vor der Ölfilterung mit Zusätzen von aktivierter Kohle im Verhältnis von Öl zu Kohle von 100:2-5 vorgereinigt wird, wobei das Öl auf eine Temperatur von 50-60° C erhitzt und diesen Temperaturen für 40-60 Minuten ausgesetzt wird, und
**dass** darauf eine einzelne Filterung ausgeführt wird.

## Revendications

1. Procédé de production d'huile de tabac à utilisation médicale, qui comprend le nettoyage des graines de tabac, le pressage et la séparation en fractions liquide et solide, la décantation de la fraction liquide obtenue avec récupération ultérieure de l'huile, suivie par une filtration de l'huile, **caractérisé en ce que** la fraction d'huile obtenue est décantée pendant 12 à 18 heures et qu'elle est, avant la filtration de l'huile, pré-nettoyée par des additions de charbon actif selon un rapport huile:charbon = 100 : 2-5, l'huile étant chauffée jusqu'à 50 à 60 °C et exposée à ces températures pendant 40 à 60 minutes ; puis une filtration unique est entreprise.
